# EUROPÄISCHE PATENTSCHRIFT

(11) **EP 1 917 933 B1**
(45) Veröffentlichungstag und Bekanntmachung des Hinweises auf die Patenterteilung: **02.03.2016**
(21) Anmeldenummer: 08002478.9
(22) Anmeldetag: 14.06.2002
(51) Int. Cl.: A61F 2/30, A61F 2/28, A61F 2/38

(54) **Implantierbare Prothese**
Implantable prosthesis
Prothèse implantable

(43) Veröffentlichungstag der Anmeldung: 07.05.2008
(62) Teilanmeldung aus: 02013153.8
(73) Patentinhaber: Baumgart, Rainer, Dipl.-Ing. Dr. med., 81479 München (DE)
(72) Erfinder: Baumgart, Rainer, Dipl.-Ing. Dr. med., 81479 München (DE)
(74) Vertreter: Sloboshanin, Sergej

(56) Entgegenhaltungen:
- EP-A- 0 490 159
- EP-A- 1 033 112
- WO-A-01/78614
- WO-A-98/46173
- DE-A- 19 604 246
- DE-A- 19 906 423

## Beschreibung

Die Erfindung betrifft eine implantierbare Prothese zum Ersatz des menschlichen Hüft- oder Kniegelenks und der benachbarten Knochenschaftanteile nach dem Oberbegriff des Patentenspruchs 1.

Bei solchen im Handel befindlichen implantierbaren Prothesen (z.B. implantcast Feinguss GmbH, Buxtehude) kann der Schaftersatzteil verschiedene Längen aufweisen oder modular aufgebaut sein. Der Schaftverankerungsteil kann verschiedene Querschnitte haben, damit er den unterschiedlichen anatomischen Markraumdimensionen angepasst werden kann. Der Schaftverankerungsteil kann dabei eine Oberfläche, die ein festes Einwachsen in den Knochen ermöglicht, aufweisen.

Künstliche Hüft- und Kniegelenke sind weit verbreitet und werden bei unterschiedlichen Indikationen eingesetzt. Wenn bei jüngeren Menschen Prothesen zum Einsatz kommen, kann es vorteilhaft sein, ein verkürztes Bein nach Implantation der Prothese zu verlängern. Insbesondere aber bei Kindern im Wachstumsalter, bei denen z.B. nach Entfernung eines Knochentumors Prothesen zum Einsatz kommen, bleibt das betroffene Bein regelmäßig im Wachstum zurück.

Bekannt sind Spezialprothesen (z.B. Fa. Stryker-Howmedica, Kiel), die eine Mechanik enthalten, mit der es möglich ist, den Prothesenkörper und damit das Bein zu verlängern, Nachteilig ist, dass der Verlängerungsvorgang z.B. durch Extrembewegungen, unter Narkose aktiviert werden muss, der Verlängerungsbedarf meist akkumuliert auf große Intervalle appliziert wird und das Verhältnis von Prothese zu Restknochen immer größer wird.

Bekannt ist ferner ein völlimplantierbarer programmierbarer elektromotorischer Distraktionsmarknagel, in dem ein Elektromotor sowie ein Untersetzungsgetriebe angeordnet sind, mit dem über einen Spindelmechanismus Zugkräfte bis 1800 N realisiert werden können. Über ein Langloch oder einen Teleskopmechanismus und eine Bolzenschraube wird die Kraft auf den Knochen übertragen, was zu einer Distraktion im Osteotomiespalt (EP 0 432 253 B1) und zusätzlich bei geeigneter Distraktionsgeschwindigkeit zu einer Knochenneubildung führt.

Die Energieeinkopplung erfolgt durch Hochfrequenz über einen externen Sender, dem ein in einem Fenster im Marknagel angeordneter Empfänger zugeordnet ist (EP 1 033 112 A2) oder über einen auf die Haut aufgelegten Sender korrespondierend zu einer subkutan liegenden Empfangsantenne, die mit dem Motor in einem Nagelende über ein Kabel verbunden ist. Eine materielle Verbindung durch die Haut zu dem Implantat ist deshalb entbehrlich - es handelt sich also um ein vollständig implantierbares System (Der Orthopäde, Springer Verlag 1999, Band 28, Heft 12, Seite 1058 bis 1065).

In der WO 01/78614 A1, auf welcher der Oberbegriff von Anspruch 1 beruht, ist eine gattungsgemäße implantierbare Prothese zum Ersatz des menschlichen Hüftgelenks und der angrenzenden Knochenschaftanteile offenbart, die ein Gelenkteil, einen Schaftverankerungsteil und einen zwischen dem Gelenkteil und dem Schaftverankerungsteil angeordneten Schaftersatzteil aufweist. Der Schaftersatzteil wird von einem gelenknahen Rohrkörper und von einem gelenkfernen Rohrkörper gebildet, die teleskopartig ineinander greifen. In dem Lumen der Rohrkörper ist ein ferngesteuerter Antrieb zum axialen Auseinanderschieben der Rohrkörper angeordnet. Außerdem weisen die Rohrkörper eine Drehsicherung auf. Der Antrieb umfasst einen in den gelenknahen Rohrkörper eingebauten Magnet, der über ein Getriebe eine Spindel antreibt, die in eine Spindelmutter am unteren Ende des gelenkfernen Rohrkörpers eingreift. Der Antrieb ist bei der Bewegung eines Patienten ständig Kräften, insbesondere Torsionskräften ausgesetzt, die im Laufe der Zeit zum Verschleiß führen. Da er mit dem gelenknahen und gelenkfernen Rohrkörper mechanisch gekoppelt ist, müssen bei Verschleiß des Antriebs auch diese ausgetauscht werden, so dass eine größere Operation erforderlich ist.

In der WO 98/46173 A1 ist eine verlängerbare Endoprothese beschrieben, die zwei Rohrkörper umfasst, die teleskopartig ineinander verschiebbar sind. Es ist eine Rücklaufsperre vorgesehen, die ein federndes Element aufweist, das in eine Rasterung einklinkt, wodurch nach einem axialen Auseinanderschieben der Rohrkörper eine Rückwärtsbewegung entgegen der Verlängerungsbewegung verhindert wird.

Die der Erfindung zugrunde liegende Aufgabe besteht darin, eine implantierbare Prothese zu schaffen, mit der ohne Verbindung nach außen durch einen integrierten Antrieb der Prothesenkörper kontinuierlich verlängert werden kann.

Diese Aufgabe wird durch eine implantierbare Prothese mit den Merkmalen des Patentanspruchs 1 gelöst. Vorteilhafte Weiterbildungen der erfindungsgemäßen Prothese sind Gegenstand der Patentansprüche 2 bis 5.

Die Drehsicherung wird vorteilhafterweise von einem Zapfen an dem einen Rohrkörper und von einem Langloch oder einer Nut an dem anderen Rohrkörper gebildet, in das bzw. die der Zapfen axial verschiebbar eingreift.

Vorzugsweise wird eine Rücklaufsperre von einer Querverzahnung in der Wand des einen Rohrkörpers und von einer stabilen Lasche an der Wand des anderen Rohrkörpers gebildet, die beim Auseinanderschieben der Rohrkörper über die Querverzahnung gleitet und eine entgegengesetzte Bewegung durch Eingriff in die Querverzahnung unterbindet, falls z.B. ein Schaden im Bereich des Antriebs auftritt.

Mit dieser Ausgestaltung der erfindungsgemäßen Prothese ist es möglich, eine beispielsweise durch Wachstum erforderlich werdende Beinlängendifferenz dadurch zu korrigieren, dass der Antrieb bedarfsgerecht in Gang gesetzt und damit die Prothese langsam und kontrolliert verlängert wird, so dass auch für das umgebende Weichgewebe und alle Leitungsstrukturen eine Längenanpassung ohne Schaden und vor allem schmerzlos und deshalb ohne Narkose möglich ist. Bei dem Verlängerungsvorgang wird das Verhältnis von Prothese zu Restknochen größer.

In weiterer Ausgestaltung der Erfindung erstreckt sich ein Kanal im Gelenkteil für eine Verbindung zwischen dem Antrieb und der Außenseite des Gelenks. Dies ermöglicht es, beispielsweise eine Empfangsantenne im Unterhautfettgewebe zu positionieren, damit sie dort von außerhalb des Gewebes über einen Sender Energie empfangen und über ein im Kanal angeordnetes Kabel dem Antrieb für seine Aktivierung zuführen kann. In dem Kanal kann auch ein Schlauch angeordnet werden, damit Aktivierungskomponenten im Subkutangewebe platziert und aktiviert werden können, beispielsweise Druck über ein Fluid auf eine Antriebskomponente innerhalb der Prothese übertragen werden kann.

Als Antrieb können ein elektromotorischer Antrieb, erforderlichenfalls mit einem zusätzliche Getriebe, ein Memorymetallantrieb, ein hydraulischer Antrieb, ein pneumatischer Antrieb oder ein piezoelektrischer Antrieb und dergleichen zum Einsatz kommen, der von außen her nach dem Prinzip Sender und Antenne oder durch Druckbeaufschlagung aktivierbar ist.

Die implantierbare Prothese nach der Erfindung eignet sich beispielsweise für die Positionierung am proximalen oder distalen Femur oder an der proximalen Tibia.

Anhand von Zeichnungen wird eine beispielsweis Ausführungsform der Erfindung näher erläutert. Es zeigen:
- Fig. 1: schematisch im Schnitt eine erfindungsgemäße Prothese im nicht verlängerten Zustand,
- Fig. 2: die Prothese von Fig. 1 im verlängerten Zustand,
- Fig. 3: schematisch und perspektivisch den einen Rohrkörper der Prothese von Fig. 1 und 2 mit dem einen Teil einer Rücklaufsperre und Verdrehsicherung und
- Fig. 4: perspektivisch den anderen Rohrkörper der Prothese von Fig. 1 und 2 mit dem anderen Teil der Rücklaufsperre und Verdrehsicherung.

Die in den Zeichnungen gezeigte implantierbare Prothese hat einen Gelenkteil 10 mit einem Schaftersatzteil 12 und einem Schaftverankerungsteil 11, der in den Markraum eines Knochenteils 18 eingesetzt ist.

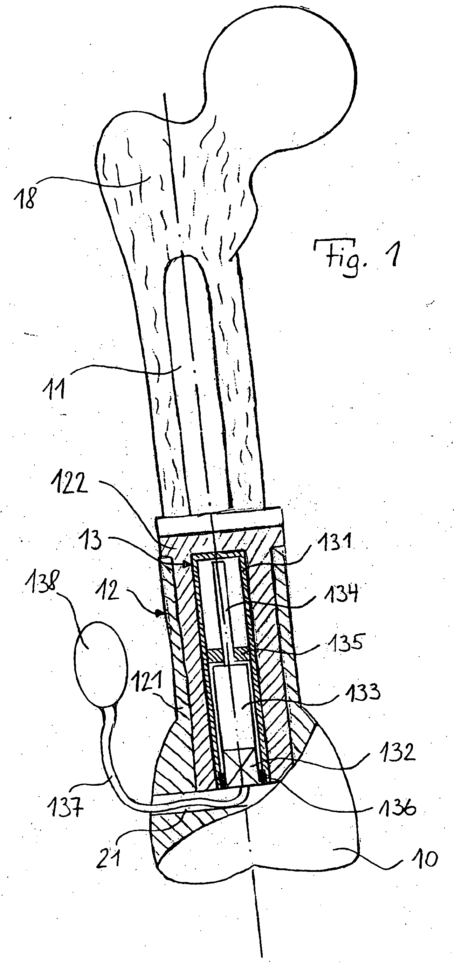

In der Ausgestaltung von Fig. 1 bis 4 besteht das Schaftersatzteil 12 aus einem am Gelenkteil 10 einstückig angeformten äußeren Rohrkörper 121, in den teleskopartig ein innerer Rohrkörper 122 eingreift, der an seinem gelenkteilfernern Ende mit dem Schaftverankerungsteil 11 verbunden ist. In dem Lumen des inneren Rohrkörpers 122 ist ein Antrieb 13 eingekapselt, der in an sich bekannter Weise ausgestaltet ist und durch Energieeinspeisung von außen aktiviert wird, wodurch die Rohrkörper zur Verlängerung der Prothese axial auseinandergeschoben werden.

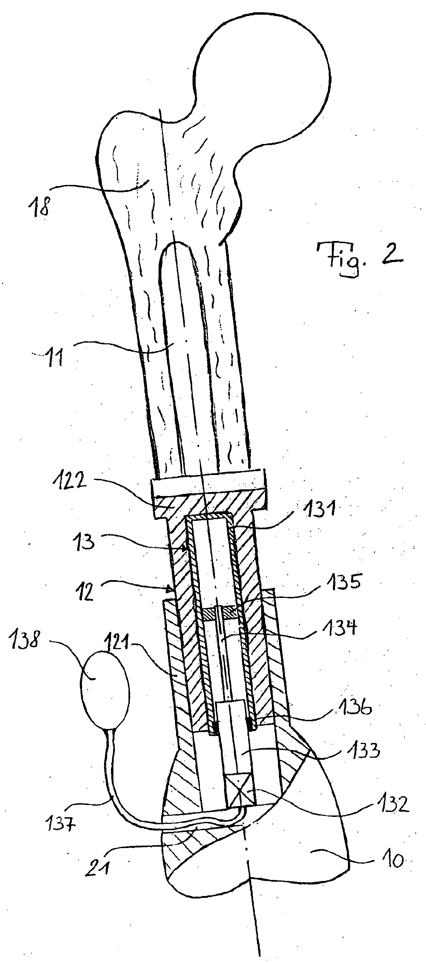

Wie in Fig. 1 und 2 gezeigt ist, hat in einer Ausgestaltung der Antrieb 13 ein Antriebsgehäuse 131, das in das Lumen des inneren Rohres 122 eingesetzt ist und beispielsweise zum Gelenkteil 10 hin offen ist. In dem Antriebsgehäuse 131 sitzt ein Motor 132, der mit einem Getriebe 133 einen Block bildet. Von dem Getriebe 133 erstreckt sich eine Gewindespindel 134 durch eine im Antriebsgehäuse 131 festgelegte Gewindemutter 135 in Gewindeeingriff mit ihr. Zur Verbesserung des Wirkungsgrades können auch spezielle Planetengetriebe zum Einsatz kommen. Zwischen dem Block und dem offenen Ende des Antriebsgehäuses 131 ist eine ein axiales Verschieben des Blocks im Antriebsgehäuse 131 zulassende Abdichtung 136 angeordnet. Der Antrieb 13 ist über ein in einem Kanal 21 im Gelenkteil 10 verlegtes Kabel 137 mit einer subkutanen Antenne 138 verbunden.

Bei Aktivierung des Motors 132 durch externe Energie wird die Gewindespindel 134 über das Getriebe 133 gedreht. Durch ihren Gewindeeingriff mit der antriebsgehäusefesten Gewindemutter 135 wird ihre Drehbewegung in eine Axialbewegung umgesetzt. Da sich der Motor 132 am Gelenkteil 10 abstützt und das Antriebsgehäuse 131 in das Lumen des inneren Rohrkörpers eingepasst ist, wird durch die axiale Abstandsvergrößerung zwischen dem Motor 132 und dem Antriebsgehäuse 131 der Rohrkörper 122 aus dem Rohrkörper 121 axial ausgefahren und dadurch die Prothese entsprechend verlängert.

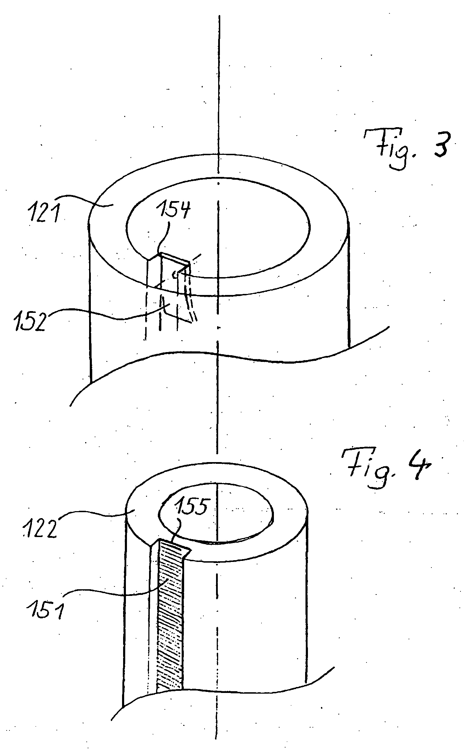

In Fig. 2 ist der Schaftersatzteil 12 in einem teilweise ausgefahrenen Zustand gezeigt. Damit eine rein axiale Verschiebung der beiden Rohrkörper 121 und 122 ohne gegenseitig Verdrehung gewährleistet ist, ist, wie in Fig. 3 und 4 gezeigt ist, an der Innenwand des äußeren Rohrkörpers 121 ein Zapfen 154 ausgebildet, der in eine axiale Nut 155 in der Außenwand des inneren Rohrkörpers 122 im Schiebesitz eingreift.

Damit bei einer Antriebsstörung eine Rückwärtsbewegung entgegen der Verlängerungsbewegung unterbunden wird, ist zwischen den beiden Rohrkörpern 121 und 122 eine in Fig. 3 und 4 gezeigte Rücklaufsperre vorgesehen. Im Boden der Nut 155 in der Außenwand des inneren Rohrkörpers 122 sind in geringen Abständen Querzähne 151 ausgebildet. An dem Zapfen 154 an der Innenwand des äußeren Rohrkörpers ist eine stabile Lasche 152 befestigt, deren freies Ende bei der Ausschiebebewegung über die Querzähne 151 gleitet, jedoch bei einer entgegengesetzten Bewegung sich mit dem Querzahn 151, mit dem sie in Kontakt steht, verhakt und so eine Rückwärtsbewegung verhindert.

In den Ausführungsformen der implantierbaren Prothese von Fig. 1 und 2 ist im Gelenkteil 10 jeweils ein Kanal 21 für die Durchführung eines Kabels oder Schlauchs vorgesehen, wodurch eine Verbindung zwischen dem Antrieb 13 und der Außenseite des Gelenkteils 10 hergestellt wird, die den Anschluss des Schlauchs oder Kabels an einen subkutanen Empfänger für Druck oder HF-Energie ermöglicht.

## Patentansprüche

1. Implantierbare Prothese zum Ersatz des menschlichen Hüft- oder Kniegelenks und der angrenzenden Knochenschaftanteile
- mit einem Gelenkteil (10),
- mit einem Schaftverankerungsteil (11) und
- mit einem zwischen dem Gelenkteil (10) und dem Schaftverankerungsteil (11) angeordneten Schaftersatzteil (12),
wobei
- der Schaftersatzteil (12) von einem gelenknahen Rohrkörper (121) und von einem gelenkfernen Rohrkörper (122) gebildet wird, die teleskopartig ineinander greifen,
- in dem Lumen der Rohrkörper (121, 122) ein ferngesteuerter Antrieb (13) zum axialen Auseinanderschieben der Rohrkörper (121, 122) angeordnet ist, und
- die Rohrkörper (121, 122) eine Drehsicherung aufweisen,
**dadurch gekennzeichnet, dass**
- der Antrieb in dem Lumen der Rohrkörper (121, 122) eingekapselt ist, wobei der Antrieb (13) ein Antriebsgehäuse (131) aufweist, das in das Lumen des inneren Rohrkörpers (122) der beiden Rohrkörper (121, 122) eingesetzt ist.

2. Implantierbare Prothese nach Anspruch 1, **dadurch gekennzeichnet, dass** die Rohrkörper (121, 122) eine Rücklaufsperre aufweisen.

3. Implantierbare Prothese nach Anspruch 1 oder 2, **dadurch gekennzeichnet, dass** die Drehsicherung von einem Zapfen (154) an dem einen Rohrkörper (121) und von einer Nut (155) an dem anderen Rohrkörper (122) gebildet wird, in das der Zapfen (154) axial verschiebbar eingreift.

4. Implantierbare Prothese nach Anspruch 2 oder 3, **dadurch gekennzeichnet**, däss die Rücklaufsperre von einer Querverzahnung (151) in der Nut (155) des einen Rohrkörpers (122) und von einer stabilen Lasche (152) an dem Zapfen (154) am anderen Rohrkörper (121) gebildet wird, die beim Auseinanderschieben der Rohrkörper (121, 122) über die Querverzahnung (151) gleitet und eine entgegengesetzte Bewegung durch Eingriff in die Verzahnung (151) unterbindet.

5. Implantierbare Prothese nach einem der vorhergehenden Ansprüche, **gekennzeichnet durch** einen Kanal (21) im Gelenkteil (10) für eine Verbindung zwischen dem Antrieb (13) und der Außenseite des Gelenkteils (10).

## Claims

1. An implantable prosthesis for replacing a human hip or knee joint and the adjoining bone shaft sections, comprising
- a joint part (10);
- a shaft anchoring part (11), and
- a shaft replacement part (12) arranged between the joint part (10) and the shaft anchoring part (11),
wherein
- the shaft replacement part (12) is formed by a tubular body (121) proximal to the joint and a tubular body (122) distal from the joint, which telescopically engage in one another,
- a remote-controlled drive (13) for axially pushing the tubular bodies (121, 122) apart is arranged in the lumen of the tubular bodies (121, 122), and
- the tubular bodies (121, 122) have an anti-rotation mechanism,
**characterized in**
- **that** the drive is encapsulated in the lumen of the tubular bodies (121, 122), wherein the drive (13) has a drive housing (131) inserted in the lumen of the inner tubular body (122) of the two tubular bodies (121, 122).

2. The implantable prosthesis according to claim 1, **characterized in that** the tubular bodies (121, 122) have a return stop.

3. The implantable prosthesis according to claim 1 or 2, **characterized in that** the anti-rotation mechanism is formed by a projection (154) at the one tubular body (121) and by a groove (155) at the other tubular body (122), in which the projection (154) engages in an axially displaceable manner.

4. The implantable prosthesis according to claim 2 or 3, **characterized in that** the return stop is formed by transverse toothing (151) in the groove (155) of the one tubular body (122) and by a solid tab (152) at the projection (154) at the other tubular body (121), which slides over the transverse toothing (151) when the tubular bodies (121, 122) are pushed apart and prevents opposite movement by engaging in the toothing (151).

5. The implantable prosthesis according to one of the preceding claims, **characterized by** a passage (21) in the joint part (10) for a connection between the drive (13) and the outside of the joint part (10).

## Revendications

1. Prothèse implantable servant à remplacer l'articulation de la hanche ou du genou de l'homme et les parties de diaphyse d'un os contiguës,
- avec une partie d'articulation (10),
- avec une partie d'ancrage (11) de diaphyse et
- avec une partie de remplacement (12) de diaphyse, placée entre la partie d'articulation (10) et la partie d'ancrage (11) de diaphyse,
dans laquelle
- la partie de remplacement (12) de diaphyse est formée par un corps tubulaire (121) proche de l'articulation et par un corps tubulaire (122) éloigné de l'articulation, s'engageant l'un dans l'autre à la manière d'un télescope,
- un entraînement (13) commandé à distance est placé dans la lumière des corps tubulaires (121, 122), servant à désengager axialement lesdits corps tubulaires (121, 122), et
- les corps tubulaires (121, 122) comportent un moyen de blocage en rotation,
**caractérisée en ce que**
- l'entraînement est encapsulé dans la lumière des corps tubulaires (121, 122), l'entraînement (13) comportant un boîtier d'entraînement (131), inséré dans la lumière du corps tubulaire intérieur (122) des deux corps tubulaires (121, 122).

2. Prothèse implantable selon la revendication 1, **caractérisée en ce que** les corps tubulaires (121, 122) comportent un dispositif anti-retour.

3. Prothèse implantable selon la revendication 1 ou 2, **caractérisée en ce que** le dispositif anti-retour est formé par un tenon (154) sur l'un des corps tubulaires (121) et par une rainure (155) sur l'autre corps tubulaire (122), rainure dans laquelle le tenon (154) s'engage avec possibilité de déplacement axial.

4. Prothèse implantable selon la revendication 2 ou 3, **caractérisée en ce que** le dispositif anti-retour est formé par une denture transversale (151) dans la rainure (155) de l'un des corps tubulaires (122) et par une languette (152) de forme stable sur le tenon (154) sur l'autre corps tubulaire (121), languette qui glisse sur la denture (151) au moment de désengager les corps tubulaires (121, 122), et empêche un mouvement en sens inverse en s'engageant dans la denture (151).

5. Prothèse implantable selon l'une des revendications précédentes, **caractérisée par** un canal (21) dans la partie d'articulation (10), pour une liaison entre l'entraînement (13) et le côté extérieur de la partie d'articulation (10).
